# EUROPEAN PATENT SPECIFICATION

(11) **EP 4 376 817 B1**
(45) Date of publication and mention of the grant of the patent: **02.07.2025**
(21) Application number: 22757589.1
(22) Date of filing: 29.07.2022
(51) Int. Cl.: A61K 9/70, A61K 31/352

(54) **TRANSMUCOSAL PATCH COMPRISING A CANNABINOID AND/OR AN OPIOID**
TRANSMUKOSALES PFLASTER MIT EINEM CANNABINOID UND/ODER EINEM OPIOID
TIMBRE TRANSMUCOSAL COMPRENANT UN CANNABINOÏDE ET/OU UN OPIOÏDE

(30) Priority: 30.07.2021 EP 21188925
(43) Date of publication of application: 05.06.2024
(73) Proprietor: Cannamedical Pharma GmbH, 50829 Köln (DE)
(72) Inventor: WAGNER, Yvonne, 53773 Hennef (DE); WINDBERGS, Maike, 61462 Königstein im Taunus (DE); PLANZ, Viktoria, 60439 Frankfurt am Main (DE); WALTHER, Alice, 60320 Frankfurt am Main (DE); FREY, Nadine, 51067 Köln (DE); SEIFERT, Anke, 52074 Aachen (DE); WALTHER, Marcel, 60433 Frankfurt am Main (DE)
(74) Representative: Wallinger Ricker Schlotter Tostmann
(86) International application number: PCT/EP2022/071416
(87) International publication number: WO 2023/006980

(56) References cited:
- WO-A1-2015/189212
- WO-A1-2018/211388
- US-A1- 2019 254 985
- SOFI HASHAM S ET AL: "Electrospun nanofibers for the delivery of active drugs through nasal, oral and vaginal mucosa: Current status and future perspectives", MATERIALS SCIENCE AND ENGINEERING C, ELSEVIER SCIENCE S.A, CH, vol. 111, 19 February 2020 (2020-02-19), XP086129843, ISSN: 0928-4931, [retrieved on 20200219], DOI: 10.1016/J.MSEC.2020.110756

## Description

### Field of the Invention

The present invention relates to a transmucosal patch comprising electrospun fibers comprising a cannabinoid or a cannabinoid and an opioid, a method for preparing same and its use as a medicament.

### Background and Object

Traditional pain relief medication includes medicines that can be bought without prescription, including paracetamol, ibuprofen and aspirin. However, these medications are known to have potential side effects which vary amongst individuals, such as those with allergies, chronic illness or that are on any other medicines.

Morphine and morphine-like drugs (for example, oxycodone, tapentadol, fentanyl and buprenorphine) are strong painkillers. They can come in different formulations such as tablets, capsules and patches and will only be prescribed after consultation with a doctor or a pain specialist as part of a long-term plan to manage pain. The dose and response will be closely monitored.

Although effective in treating pain in patients, the above medications are often accompanied by unpleasant side effects as mentioned above, and a risk of overdosing. It would be beneficial to provide a pain relief medication for use in a patient that has fewer side effects or risks as seen in traditional medication, whilst being highly effective in treating pain.

Measurable changes to serotonin, noradrenaline and dopamine levels occur in the brains of people experiencing a severe anxiety event, and thus some types of antidepressant medication, which correct the imbalance of neurotransmitters in the brain, are effective in the treatment of severe anxiety, even where unrelated to symptoms of depression.

Antidepressants can improve mood, but they do not provide a long-term solution. Side effects are often experienced, and the risks and benefits must be considered. Common side effects of antidepressant medication can include nausea, headaches, anxiety, sweating, dizziness, agitation, weight gain, dry mouth and sexual difficulties.

The therapeutic use of cannabinoids for e.g., the treatment or prophylaxis of pain has already been proven as quite successfully. So far, extracts with varying compositions of the active ingredients have been predominantly used in the form of magistral recipes.

WO 2018/211388 A1 describes sublingual muco-adhesive cannabinoid compositions in the form of sublingual tablets or films. So far, only one finished medicinal product comprising a combination of tetrahydrocannabinol (THC) and cannabidiol (CBD) as an oral mucosal spray has gained marketing authorization as a pharmaceutical and is marketed in Germany under the tradename Sativex^{®}. However, since most of the applied dosage is swallowed, the bioavailability via the gastrointestinal tract is strongly influenced resulting in a high inter- and intra-individual variation in efficacy. In general, only a portion of the administered dosage of THC and CBD are pharmaceutically active within the patient.

EP 2 283 832 relates to a cannabinoid based medicinal extract comprising one or more cannabinoids in combination with volatile oils and is used for treating nausea and vomiting.

EP 1 901 734 relates to a pharmaceutical composition comprising THC in the crystalline form and a pharmaceutically acceptable carrier and its use as medicament. As indications to be treated pain, emesis, loss of appetite or weight loss are the subject of said patent.

EP 3 681 525 relates to a pharmaceutical composition comprising CBD and THC in a ratio of CBD : THC from about 0.1 : 5 to about 5 : 0.1, which generally comprises one or more pharmaceutically acceptable carriers, diluents, excipients, excipients, or any combination thereof. As a carrier, several oils, such as olive oil, hemp oil, sesame oil, coconut oil, etc. are disclosed. This composition is predominantly for the treatment of symptoms of autism, such as reduced sociability, tantrums, poor use of language, etc.

EP 3 525 823 discloses a composition for the treatment of neurodegenerative diseases, wherein said composition comprises a mixture of two cannabinoids and a pharmaceutically acceptable carrier or diluent, wherein said composition is in the form of an oil, emulsion, gel, or an aerosol.

EP 3 171 871 relates to a pharmaceutical composition in the form of a solid dosage form for sublingual or buccal administration. The composition comprises a cannabinoid and a pharmaceutically acceptable solvent into which the cannabinoid is solvated, and a pharmaceutically acceptable adsorbent onto which the solvated cannabinoid is adsorbed. The adsorbent is predominantly selected from silica, microcrystalline cellulose, cellulose, silicified microcrystalline cellulose, clay, talc, starch, pre-gelatinized starch, calcium carbonate, dicalcium phosphate, magnesium carbonate, and mixtures thereof.

WO 2005/120478 relates to a pharmaceutical composition comprising a combination of CBD and THC for use as a disease modifying treatment of rheumatoid arthritis. The ratio of CBD : THC is defined as being substantially 1 : 1 and the dose taken by the patient is in the range of 5 to 25 mg of each cannabinoid per day sublingually or buccally.

EP 3 641 726 and EP 3 641 727 relate to a pouch designated for administration of an active ingredient in the oral cavity, the pouch containing a matrix composition comprising a combination of an amount of one or more cannabinoids and a water-insoluble composition. The water-insoluble composition is defined as comprising cellulose. These references also disclose the use of said pouches for the alleviation of pain, in particular neurotic pain and cancer-related pain.

Electrospinning is a versatile method for preparing fibers of biocompatible polymers that may also be used for pharmaceutical application. In this regard, reference is made to European patent application EP 2020 0785.2, filed with the European Patent Office on October 8, 2020, and the prior art disclosed therein. Further reference is made to US 2019/254985 A1 which describes electrospun fibers containing: i) a first and a second hydrophilic fiber-forming polymer that is soluble in a hydrophilic solvent, ii) a bioadhesive substance that is slightly soluble in said hydrophilic solvent, iv) a drug substance, and to WO 2015/189212 A1 which describes electrospun fibers comprising i) a hydrophilic polymer that is soluble in a first solvent, ii) a bioadhesive substance that is slightly soluble in said first solvent, iii) optionally, a drug substance.

A review regarding electrospinning has been published under the title "Electrospinning: A Fascinating method for the Preparation of Ultrathin Fibers" in Angew. Chem. Ind. Ed. 2007, 46, 5670-5703. With regard to pharmaceutical applications of electrospun nanofibers reference is made to a publication authored by Windbergs et al. under the title "Functional electrospun fibers for the treatment of human skin wounds" and published in European Journal of Pharmaceutics and Biopharmaceutics 119 (2017) 283-299, a publication by Shitole et al. in Asian J. Pharm. Tech. 2020; 10(3); 187-201 and to a publication authored by Sofi et al. with the title "Electrospun nanofibers for the delivery of active drugs through nasal, oral and vaginal mucosa: Current status and future perspectives" and published in Material Science & Engineering 2020; C111(110756); 1-20

A common problem associated with administration via the buccal route is swallowing the dose due to the continuous secretion of saliva in the oral cavity. This contributes to the above-discussed and often observed pharmacokinetics variability of oral cannabinoid sprays or aerosols. For optimum drug delivery, the buccal and sublingual dosage form must remain in contact with oral mucosa for a time sufficient to allow for the absorption of the cannabinoid or opioid. In particular, it is necessary to prevent that the dosage form is washed away by saliva into the gastrointestinal tract. At the same time, the rate of disintegration or dissolution of the dosage form must not be so slow as to cause discomfort or inconvenience for the patient. Additionally, suitable buccal and sublingual dosage forms should be small in size and designated so that the shape avoids discomfort to the patient during use. Most importantly, the formulation must be designed so that the cannabinoid is delivered such that its transmucosal absorption is optimized.

It would therefore be desirable to provide a cannabis-based medicine that is effective in the treatment and prophylaxis of various conditions including but not limited to reduce and/or alleviate the symptom burden, such as pain and anxiety, of severe diseases, such as cancer or ALS (amyotrophic lateral sclerosis), while not causing or minimizing the side effects in relation to cannabis use, such as impairment of cognitive functions.

**Summary of the Invention**The present invention relates to a transmucosal patch comprising electrospun fibers comprising a biocompatible polymer and a therapeutically effective amount of a pharmaceutical active ingredient, wherein the active pharmaceutical ingredient comprises a cannabinoid or a cannabinoid and an opioid.

The patch may be an oral transmucosal patch, in particular a buccal or sublingual transmucosal patch.

The patch according to any of the above embodiments, wherein the active pharmaceutical ingredient is a cannabinoid or a cannabinoid and an opioid.

The patch according to any of the above embodiments, which comprises an additional active pharmaceutical ingredient, which is not an opioid or a cannabinoid.

The patch according to any of the above embodiments, wherein the active pharmaceutical ingredient comprises THC, which is present in an amount of 1 to 50, or 2 to 30, or 2.5 to 25, or 5 to 20, or 10 to 15 mg, preferably 2.5, 5 or 10 mg.

The patch according to any of the above embodiments, wherein the active pharmaceutical ingredient is present in an amount of from 0,125 to 25 mg/cm².

The patch according to any of the above embodiments, wherein the active pharmaceutical ingredient comprises at least one opioid selected from buprenorphine, codeine, fentanyl, hydromorphone, hydrocodone, oxycodone, dihydrocodeine, dihydromorphine, morphine, tapentadol, tramadol, oxymorphone, pharmaceutically acceptable salts thereof, and combination of two or more thereof.

The patch according to any of the above embodiments, wherein the active pharmaceutical ingredient is at least one cannabinoid.

The patch according to any of the above embodiments, wherein the active pharmaceutical ingredient is at least one cannabinoid selected from cannabidiol (CBD), cannabichromene (CBC), cannabigerol (CBG), delta-9-tetrahydrocannabinol (THC), cannabinol (CBN), THCA (tetrahydrocannabinolic acid), CBDA (cannabidiolic acid), CBN (cannabinol), CBG (cannabigerol), CBC (cannabichromene), CBL (cannabicyclol), CBV (cannabivarin), THCV (tetrahydrocannabivarin), CBDV (cannabidivarin), CBCV (cannabichromevarin), CBGV (cannabigerovarin), CBGM (cannabigerol monomethyl ether), CBE (cannabielsoin), and CBT (cannabicitran) or a combination of two or more thereof.

The patch according to any of the above embodiments, wherein the active pharmaceutical ingredient is at least one cannabinoid selected from cannabidiol (CBD), cannabichromene (CBC), cannabigerol (CBG), delta-9-tetrahydrocannabinol (THC), and cannabinol (CBN), or a combination of two or more thereof.

The patch according to any of the above embodiments, wherein the active pharmaceutical ingredient comprises a combination of THC and CBD, or is a combination of THC and CBD.

The patch according to any of the above embodiments, wherein the ratio between THC and CBD is from 100:1 to 1:100, or 50:1 to 1:50, or 20:1 to 1:20, or 10:1 to 1:10, or 5:1 to 1:5, or 4:1 to 1:4, or 3:1 to 1:3, or 2:1 to 1:2, or 1:1, in particular 5:1, or 4:1, or 3:1, or 2:1, or 1:1.

The patch according to any of the above embodiments, wherein the ratio between THC and CBD is 2:1 to 1:2, in particular 1:1.

The patch according to any of the above embodiments, wherein the active pharmaceutical ingredient is immediately released.

The patch according to any of the above embodiments, wherein the active pharmaceutical ingredient is released in a controlled release manner.

The patch according to any of the above embodiments, wherein a part of the active pharmaceutical ingredient is immediately released and the remainder of the active pharmaceutical ingredient is released in a controlled release manner.

The patch according to any of the above embodiments, wherein the active pharmaceutical ingredient is released within 5 seconds to 5 hours, 30 seconds to 5 hours, or 15 minutes to 5 hours, or 25 minutes to 4 hours, or 30 minutes to three hours, or 60 minutes to two hours, preferably within 30 minutes to two hours.

The patch according to any of the above embodiments comprising pharmaceutically acceptable excipients.

The patch according to any of the above embodiments, wherein the biocompatible polymer is partly or completely dissolvable in contact with saliva.

The patch according to any of the above embodiments, wherein the biocompatible polymer stays intact in contact with saliva.

The patch according to any of the above embodiments, wherein the biocompatible polymer is of natural or synthetic nature.

The patch according to any of the above embodiments, wherein the biocompatible polymer is selected from the group consisting of chitosan, dextrin, alginates, poly(glycolic acid), poly(lactic acid), poly(lactide-co-glycolic acid) (PLGA), hyaluronic acid, CMC, poly(hydroxyethyl methacrylates), polyethylene oxide, polyvinylpyrrolidone, polyvinyl alcohol, poly(acrylic acids).

The patch according to any of the above embodiments, where the electrospun fibers have a core-shell structure with the polymer forming the shell and the active pharmaceutical ingredient forming the core.

The patch according to any of the above embodiments, wherein the patch is self-adhering to the mucosa.

The patch according to any of the above embodiments, which is in the form of a square, a rectangle, an oval, round-edged squares or rectangle, a circle.

The patch according to any of the above embodiments for use as a medicament.

The patch according to any of the above embodiments for use in the treatment or prophylaxis of the symptom burden in a patient suffering from a symptoms' generating disease, in particular a severe chronic disease such as cancer or ALS.

The patch according to any of the above embodiments, wherein the symptoms to be treated are selected from pain, depression, restless leg(s), nausea, constipation, vomiting, decreased or loss of appetite, a sleep disorder, pruritus, anxiety, fatigue, dyspnea, numbness in extremity, dizziness, confusion, memory problems, euphoria, restlessness, somnolence, vomiting, abdominal pain, diarrhea, tachycardia, chest pain, itching, dry mouth, respiratory problems, cramps, spasms, stress, overall reduced activity, reduced quality of life and recurrent infections, inflammation, and a combination of two or more thereof.

The patch according to any of the above embodiments, wherein the symptom is selected from anxiety, pain, overall reduced activity and/or a reduced quality of life.

The patch according to any of the above embodiments for use in the treatment or prophylaxis of pain.

The patch according to any of the above embodiments, wherein the pain is resulting from inflammation, including inflammation from multiple sclerosis, Alzheimer disease or other neurodegenerative diseases, Parkinson's disease, motor neuron diseases, neurodegenerative disease, or traumatic brain injury.

The patch according to any of the above embodiments, wherein the pain is acute or chronic pain including pain from motor neuron diseases, multiple sclerosis, Fibromyalgia, cancer, ALS and peripheral neuropathy.

The patch according to any of the above embodiments, wherein the pain is back pain, including upper/neck, low, shoulder and cervical pain, neuropathic pain, cancer- or ALS-related pain.

In one embodiment, neck and/or cervical pain are treated.

In a further embodiment neuropathic pain is treated.

The patch according to any of the above embodiments for use in the treatment or prophylaxis of pain in relation to diseases or conditions resulting in pain as a side effect.

The patch according to any of the above embodiments, wherein said treatment is a second or third line or adjunctive/add-on treatment of cancer.

The patch according to any of the above embodiments, wherein said treatment is a second or third line or adjunctive/add-on treatment of ALS.

The patch according to any of the above embodiments, wherein during the prophylaxis or treatment a daily dosage of 1 to 50 mg, or 2 to 40 mg, or 2.5 to 30 mg, or 5 to 25 mg, or 10 to 20 mg of THC is administered.

The present invention furthermore relates to a process for preparing a patch according to any of the preceding embodiments comprising the following steps (a) to (c):
(a) Providing a solution of a biocompatible polymer;
(b) Providing a liquid comprising the active pharmaceutical ingredient;
(c) Electrospinning said solution of a biocompatible polymer to produce a fiber comprising said polymer.

The process according to the above embodiment, wherein said step (c) comprising the coaxial electrospinning of said solution comprising the biocompatible polymer and said liquid comprising the active pharmaceutical ingredient, such that core-shell fibers are formed with the biocompatible polymer forming the shell and the active pharmaceutical ingredient forming the core.

In one embodiment, further excipients are present in said solution and/or said liquid.

The process according to the above embodiments, wherein further excipients are present in said solution and/or said liquid, preferably said further excipients are selected from the group consisting of cosolvents, emulsifiers, agents increasing the electrical conductivity, buffers, penetration enhancers and viscosity-modifying agents.

### Detailed Description

### Introduction

The pharmaceutical dosage forms according to the present invention are transmucosal patches, which allow the local mucosal application of a cannabinoid or a cannabinoid and an opioid. The patch comprises as active pharmaceutical ingredients in particular a therapeutically effective amount of a combination of THC and CBD. The patch allows predominantly the administration of these active pharmaceutical ingredients via the oral mucosa. The patches on the basis of electrospun biocompatible polymer fibers adhere to the mucosa and release the active ingredients directly to the same, resulting in a high and uniform delivery of active ingredients into the patient's blood stream by surpassing the decomposition of the active ingredients via the liver.

The delivery of the active pharmaceutical ingredient may be via immediate release or in a controlled release manner. In a further embodiment, the patch may be such that part of the active pharmaceutical ingredient may be delivered via immediate release, and the remainder being delivered in a controlled release manner.

It has been discovered that the transmucosal patch of the present invention improves (oral) bioavailability with fast onset of cannabinoid action compared to other prior art forms for (oral) delivery of cannabinoids. It thus provides an unmet medical need for a convenient, safe, fast acting, oral dosage form which provides accurate and timely cannabinoid delivery with increased oral bioavailability as it avoids swallowing the dosage and subsequent high first-pass metabolism associated with gastrointestinal absorption of cannabinoids.

In contrast to most of the orally administered dosage forms the application via a patch does not require any intake of water. The patch can be applied discretely in basically any situation and circumstance. The composition and the diameter and size of the individual fibers in the patch control the release kinetics of the active ingredient. In certain embodiments, the transmucosal patch stays in the oral cavity, since it dissolves under the influence of saliva over a predetermined time. The size of the patch can easily be adjusted by simply cutting the same.

Unless otherwise defined, all technical and scientific terms used herein have the same meaning as commonly understood by one of ordinary skill in the art to which this disclosure belongs.

In particular, it is noteworthy that the use of the undefined article "a" or "an" means "one or more". Thus, the term "a active pharmaceutical ingredient" always includes the incorporation of "one" and "more than one" active ingredient.

### Transmucosal patch

The term *"transmucosal"* means that the active ingredient within the claimed patch is entering the patient's bloodstream through or across a mucous membrane. The term *"transmucosal"* in its broadest meaning encompasses the delivery of an active pharmaceutical ingredient via the mucosal linings of the nasal, rectal, vaginal, ocular, and oral cavity. In particular, the patch is an oral transmucosal patch, which is typically applied buccally or sublingual.

The patch may be self-adhering to the mucosa, or may comprise an adhesive component, which is constituted by a biocompatible adhesive.

The patch may be in any form suitable to be applied to the mucosa. Typical forms are square, rectangular, oval, round-edged square or rectangle, or circle. The patch may be packaged individually in one of the above-described shapes ready-to-use for the patient to be treated. Alternatively, it may be packaged in "stock-form" form with the possibility for the patient to cut out an appropriate shape and size.

### Active pharmaceutical ingredient

The term "active pharmaceutical ingredient" as used herein is used interchangeably with the term "active ingredient" or API and refers to an agent or a mixture of agents that causes a desired therapeutic effect. The active ingredient within this disclosure comprises a cannabinoid or a cannabinoid and an opioid.

In one embodiment, the active ingredient is a cannabinoid or a cannabinoid and an opioid or a combination of two or more thereof.

In one embodiment, the opioid is at least one opioid.

Examples of useful opioids include, but are not limited to, alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, fentanyl, heroin, hydrocodone, hydromorphone, hydroxypethidine, isomethadone, ketobemidone, levorphanol, levomethadone, levophenacylmorphan, lofentanil, meperidine, meptazinol, metazocine, methadone, metopon, morphine, myrophine, nalbuphine, narceine, nicomorphine, norlevorphanol, normethadone, nalorphine, normorphine, norpipanone, opium, oxycodone, oxymorphone, papaveretum, pentazocine, pethidine, phenadoxone, phenomorphan, phenazocine, phenoperidine, piminodine, piritramide, proheptazine, promedol, properidine, propiram, propoxyphene, sufentanil, tilidine, tapentadol, tramadol, pharmaceutically acceptable salts thereof, and mixtures thereof.

In certain embodiments, the opioid is selected from buprenorphine, codeine, fentanyl, hydromorphone, hydrocodone, oxycodone, dihydrocodeine, dihydromorphine, morphine, tapentadol, tramadol, oxymorphone, pharmaceutically acceptable salts thereof, and mixtures thereof.

The term "cannabinoid" as used herein includes chemical compounds that are present in cannabis plants as well as synthetic cannabinoids. So far, more than 110 compounds have been found in the cannabis plant, which are all to be subsumed under the term "cannabinoid" as used herein. Cannabinoids may be endocannabinoids (produced in the body of animals), phytocannabinoids (produced in cannabis and some other plants), and synthetically manufactured cannabinoids.

The cannabinoids may be included in its free form, or in the form of a salt; an acid addition salt of an ester; an amide; an enantiomer; an isomer; a tautomer; a prodrug; different isomeric forms (for example, enantiomers and diastereoisomers), both in pure form and as a mixture, including racemic mixtures. Cannabinoids include compounds (such as THC) that have high affinity for the cannabinoid receptor (for example Ki < 250 nM), and compounds that do not have significant affinity for the cannabinoid receptor (such as cannabidiol (CBD)). Cannabinoids also include compounds that have a characteristic dibenzopyran ring structure (of the type seen in THC) and cannabinoids which do not possess a pyran ring (such as cannabidiol (CBD)).

The term "cannabinoid" is also meant to encompass derivatives that are produced from another compound of similar structure by the replacement of, e.g., substitution of one atom, molecule or group by another, e.g., cannabidiol (CBD), delta-9-tetrahydrocannabinol (THC). Other cannabinoids include dimethyl heptylpentyl cannabidiol (DMHP-CBD), 6,12-dihydro-6-hydroxy-cannabidiol (see, e.g., US 5,227,537); (3S,4R)-7-hydroxy-A6-tetrahydrocannabinol homologs and derivatives (described in U.S. 4,876,276); (+)-4-[4-DMH-2,6-diacetoxy- phenyl]-2-carboxy-6,6-dimethylbicyclo[3.1.1]hept-2-en, and other 4-phenylpinene derivatives (see, e.g., US 5,434,295), and cannabidiol (-)(CBD) analogs such as (-)CBD-monomethylether, (-)CBD dimethyl ether; (-)CBD diacetate; (-)3'-acetyl-CBD monoacetate; and ±AFI I (see, e.g., Consroe et al. (1981) J. Clin. Pharmacol. 21 :428S-436S). cannaichromene (CBC), cannabichromenic acid (CBCV), cannabidiolic acid (CBDA), cannabidivarin (CBDV), cannabigerol (CBG), cannabigerol variant (CBGV), cannabicyclol (CBL), cannabicyclol (CBN), tetrahydrocannabivarin (THCV), tetrahydrocannabivarinic acid (THCVA), cannabitriol (CBO), cannabinol propyl variant (CBNV), and tetrahydrocannabinolic acid (THCA). Many other useful cannabinoids are disclosed in Agurell et al. (1986) Pharmacol. Rev. 38:31-43.

The term cannabinoid also includes prodrugs of cannabinoids, as well as pharmaceutically acceptable salts, metabolites, such as 11-OH-THC, and complexes of cannabinoids.

In one embodiment, the cannabinoid is at least one cannabinoid selected from cannabidiol (CBD), cannabichromene (CBC), cannabigerol (CBG), delta-9-tetrahydrocannabinol (THC), cannabinol (CBN), THCA (tetrahydrocannabinolic acid), CBDA (cannabidiolic acid), CBN (cannabinol), CBG (cannabigerol), CBC (cannabichromene), CBL (cannabicyclol), CBV (cannabivarin), THCV (tetrahydrocannabivarin), CBDV (cannabidivarin), CBCV (cannabichromevarin), CBGV (cannabigerovarin), CBGM (cannabigerol monomethyl ether), CBE (cannabielsoin), and CBT (cannabicitran) or a combination of two or more thereof.

In one embodiment, the active pharmaceutical ingredient may be at least one cannabinoid selected from cannabidiol (CBD), cannabichromene (CBC), cannabigerol (CBG), delta-9-tetrahydrocannabinol (THC), and cannabinol (CBN).

In one embodiment, the active pharmaceutical ingredient is a combination of THC and CBD.

The ratio between THC and CBD may be from 20:1 to 1:20, or from 10:1 to 1:10, or from 5:1 to 1:5, or from 4:1 to 1:4, or from 3:1 to 1:3, or from 2:1 to 1:2, or 4:1 or 3:1 or 2:1 or 1:1.

More generally speaking, the active pharmaceutical ingredient is THC and is present in an amount of 1 to 50, or 2 to 30, or 2.5 to 25, or 5 to 20, or 10 to 15 mg, preferably 2.5, 5 or 10 mg in the patch.

In one embodiment the active pharmaceutical ingredient is present in an amount of from 0,125 to 25 mg/cm² of the patch.

In one embodiment, the active pharmaceutical ingredient is released within 5 seconds to 5 hours, 30 seconds to 5 hours, or 15 minutes to 5 hours, or 25 minutes to 4 hours, or 30 minutes to three hours, or 60 minutes to two hours, preferably within 30 minutes to two hours.

### Electrospun fibers comprising a biocompatible polymer

The patch according to the present invention comprises a biocompatible polymer in the form of electrospun fibers. In certain embodiments said polymer is mucoadhesive.

Mucoadhesion (or muco-adhesion) is generally understood to define the ability of a biological or synthetic material to "stick" to a mucous membrane, resulting in bioadhesion of the material to the tissue for a protracted period of time. For a material to be mucoadhesive, it must interact with mucus, which is a highly hydrated, viscous hydrogel layer protecting the mucosa.

Factors to be considered for use of a biocompatible polymer, particularly for the shell of the fiber, include and are based on biocompatibility, hydrophilicity, hydrophobicity, dissolution, solubility, and/or scaffold stability of the polymer, as well as the final shell material as used. Of course, the material must be suitable for electrospinning. Preferred are biocompatible polymers that can act as hydrogen bond donors, facilitate the interaction with water, and lead to water absorption, and/or also retain structural integrity.

Of course, the biocompatible polymer needs to be electro spinnable. In addition, it needs to be compatible with the cannabinoid or the cannabinoid and opioid.

The biocompatible polymer can be either dissolvable in contact with saliva or will not dissolve due to the contact with the same.

Examples of preferred biocompatible polymers are selected from polycaprolactone (PCL), chitosan, alginate, poly(acrylic acids), CMC, polyethylene oxide (PEO)/polyethylene glycol (PEG), and/or poly(D,L-lactic-co-glycolic) acid (PLGA) and biocompatible derivatives thereof. These derivatives are known to the person of skill, and can be taken from the respective literature (e.g. for PLGA, see Sah H, Thoma LA, Desu HR., Sah E, Wood GC. Concepts and practices used to develop functional PLGA-based nanoparticulate systems. Int. J Nanomedicine. 2013; 8:747-765. doi:102147/11N.540579; Snejdrova E, Podzimek S, Martiska J, Holas 0, Dittrich M. Branched PLGA derivatives with tailored drug delivery properties. Acta Pharm. 2020; 70(1):63-75. doi:102478/acph-2020-0011. Furthermore, HPMC, HMC, and polyvinylpyrollidone may be used.

Within the ambit of the present invention, several electrospinning methods are available for preparing the polymer fiber, typically together with the active ingredient.

This means that the polymer fiber may be prepared by blend electrospinning. During blend electrospinning a composition/blend of the active pharmaceutical ingredient, here a cannabinoid or a cannabinoid and an opioid, optionally together with an additional pharmaceutical ingredient, in the following also referred to as "API", is dissolved or dispersed in the electrospinning polymeric solution and the liquid spun for fiber generation. This results in electrospun polymeric fibers with the API being uniformly dispersed therein.

An analogous form of electrospinning is the so-called emulsion electrospinning. This form of electrospinning is based on an emulsion. Typically, the API together with a surfactant forms one phase and forms together with an immiscible solvent an emulsion, which is mixed with a suitable polymer and electrospun to produce the respective fibers.

Alternatively, the polymer fiber may be prepared by co-axial electrospinning. The co-axial electrospinning uses generally two or more nozzles that connect to the high voltage. E.g., two different solutions and/or emulsions and/or suspensions, namely the polymer solution and the solution or emulsion or suspension comprising the API, are applied via concentric nozzles. In one embodiment, the solution/emulsion/suspension comprising the API is spun through the inner jet/nozzle and the polymer is spun through the outer jet/nozzle. Conclusively, a core-shell structure results, with the API forming the core being covered by the polymer, which represents the shell. A polymer fiber prepared by coaxial electrospinning is particularly suitable for a controlled-release administration of the API.

For further details regarding the various embodiments of electrospinning and ultimately obtaining the fibers to be used in the patch according to the invention, particular reference is made to the references already cited in the introductory part of the present application.

The solution/emulsion/suspension comprising the API and the polymer, respectively, may comprise excipients known in the art, such as buffers or surfactants.

In the context of the present invention, any suitable solvent may be used to dissolve the biocompatible polymer(s). The solvent needs to be compatible with the biocompatible polymer(s) and must be suitable for electrospinning process. Also, a mixture of suitable solvents can be used, such as, for example, alcohols, such as ethanol, and chloroform.

### Additional active pharmaceutical ingredients (additional APIs)

The patch of the present invention may further include one or more additional (or second) active pharmaceutical ingredients that may or may not act synergistically with the cannabinoid or the cannabinoid and the opioid present in said patch. Said additional API is neither a cannabinoid nor an opioid.

The additional API can be a non-opioid analgesic, a non-steroidal antiinflammatory agent, an antimigraine agent, a Cox-II inhibitor, a β-adrenergic blocker, an anticonvulsant, an antidepressant, an anticancer agent, an agent for treating addictive disorder, an agent for treating Parkinson's disease and parkinsonism, an agent for treating anxiety, an agent for treating epilepsy, an agent for treating a seizure, an agent for treating a stroke, an agent for treating a pruritic condition, an agent for treating psychosis, an agent for treating ALS, an agent for treating a cognitive disorder, an agent for treating a migraine, an agent for treating vomiting, an agent for treating dyskinesia, an agent for treating depression, Alzheimer disease, other neurodegenerative diseases, inflammatory diseases, multiple sclerosis, Crohn's disease, a Na-channel blocking agent, a Ca-channel blocking agent, anticonvulsiva or a mixture thereof.

Examples of useful non-opioid analgesics include non-steroidal antiinflammatory agents, such as aspirin, ibuprofen, diclofenac, naproxen, benoxaprofen, flurbiprofen, fenoprofen, flubufen, ketoprofen, indoprofen, metamizole, novaminsulfon, piroprofen, carprofen, oxaprozin, pramoprofen, muroprofen, trioxaprofen, suprofen, aminoprofen, tiaprofenic acid, fluprofen, bucloxic acid, indomethacin, sulindac, tolmetin, zomepirac, tiopinac, zidometacin, acemetacin, fentiazac, clidanac, oxpinac, mefenamic acid, meclofenamic acid, flufenamic acid, niflumic acid, tolfenamic acid, diflurisal, flufenisal, piroxicam, sudoxicam, isoxicam, and pharmaceutically acceptable salts thereof, and mixtures thereof.

Examples of anticonvulsive drugs are pregabalin, gabapentin, primidon, felbamat, topiramat, diazepam, lorazepam, phenobarbital, clonazepam and clobazam.

Examples of other suitable non-opioid analgesics include the following, nonlimiting, chemical classes of analgesic, antipyretic, nonsteroidal antiinflammatory drugs: salicylic acid derivatives, including aspirin, sodium salicylate, choline magnesium trisalicylate, salsalate, diflunisal, acetylsalicylic acid, sulfasalazine, and olsalazin; para aminophenol derivatives including acetaminophen and phenacetin; indole and indene acetic acids, including indomethacin, sulindac, and etodolac; heteroaryl acetic acids, including tolmetin, diclofenac, and ketorolac; anthranilic acids (fenamates), including mefenamic acid, and meclofenamic acid; enolic acids, including oxicams (piroxicam, tenoxicam), and pyrazolidinediones (phenylbutazone, oxyphenthartazone); and alkanones, including nabumetone.

For a more detailed description of the NSAIDs, see Paul A. Insel, Analgesic Antipyretic and Antiinflammatory Agents and Drugs Employed in the Treatment of Gout, in Goodman & Gilman's The Pharmacological Basis of Therapeutics 617-57 (Perry B. Molinhoff and Raymond W. Ruddon eds., 9th ed 1996) and Glen R. Hanson, Analgesic, Antipyretic and Anti Inflammatory Drugs in Remington: The Science and Practice of Pharmacy Vol II 1196-1221 (A.R. Gennaro ed. 19th ed. 1995 ). Suitable Cox-II inhibitors and 5-lipoxygenase inhibitors, as well as combinations thereof, are described in U.S. Patent No. 6,136,839. Examples of useful Cox-II inhibitors include, but are not limited to, rofecoxib and celecoxib.

### Pharmaceutically acceptable excipients

The term "pharmaceutically acceptable" means that an excipient has been approved by an official regulatory agency or listed in a generally recognized pharmacopeia for use in animals, and more particularly in humans.

Useful water-soluble diluents must be pharmaceutically acceptable. Useful diluents include, but are not limited to, sugars, polyols, saccharides, polysaccharides, dextrate, dextrins, dextrose, fructose (ADVANTOSE FS 95), lactitol (FINLAC DC), lactose, erythritol, maltose, isomalts, maltitol, a maltodextrin, a polydextrose, trehalose, mannitol (PEARLITOL 300 DC, PEARLITOL 400 DC, PEARLITOL 500 DC, MA OGEM 2080, MA OGEM EZ, PARTEKM100, PARTECK M200, PARTECK M300), a polyethylene glycol, sorbitol (PARTECK SI 150, PARTECK SI 400, PARTECK SI 450), sucrose, xylitol and mixtures thereof. One exemplary diluent is mannitol.

Other useful excipients that can be comprised in the patch include, but are not limited to, a colorant, a flavoring, a coating agent, a sweetening agent, a viscosity agent, a controlled-release agent, a mucoadhesive, a dissolution enhancer, a buffering agent (e.g., phosphate, carbonate, tartrate, borate, citrate, acetate, and malate buffers), a penetration enhancer and combinations thereof.

### Conditions to be treated

It the broadest definition, the above-described patch may be used as a medicament in a patient.

The patient is an animal or a human being.

The patch may be used in the treatment or prophylaxis of the symptom burden in a patient suffering from a symptoms' generating disease, in particular a severe chronic disease such as ALS or cancer.

The symptoms to be treated include, but are not limited to, pain, depression, restless legs, nausea, constipation, vomiting, decreased or loss of appetite, a sleep disorder, pruritus, anxiety, fatigue, dyspnea, numbness in extremity, dizziness, confusion, memory problems, euphoria, restlessness, somnolence, vomiting, abdominal pain, diarrhea, tachycardia, chest pain, itching, dry mouth, cramps, spasms, respiratory problems, overall reduced activity, reduced quality of life and recurrent infections, inflammation, in particular the symptom is selected from anxiety, pain, overall reduced activity and/or reduced quality of life.

The patch may also be used for the treatment or prophylaxis of pain. The pain may result from inflammation, including inflammation from multiple sclerosis, Alzheimer disease or other neurodegenerative diseases, Parkinson disease, motor neuron disease and traumatic brain injury.

The pain may be acute or chronic pain including pain from motor neuron disease, multiple sclerosis, Fibromyalgia, cancer, ALS and peripheral neuropathy.

The pain is or may include back pain, including upper/neck, shoulder, cervical and lower back pain, neuropathic pain, cancer-or ALS-related pain.

The patch may also be used in the treatment or prophylaxis of pain in relation to diseases or conditions resulting in pain as a side effect.

The patch may also be used in a second or third line or adjunctive/add-on treatment of cancer or ALS.

The terms "treatment", "treating", and the like are used herein to generally mean obtaining a desired pharmacologic and/or physiologic effect. The effect may be prophylactic, in terms of completely or partially preventing a disease, condition, or symptoms thereof, and/or may be therapeutic in terms of a partial or complete cure for a disease or condition and/or adverse effect, such as a symptom, attributable to the disease or condition. "Treatment" as used herein covers any treatment of a disease or condition of a mammal, particularly a human, and includes: (a) preventing the disease or condition from occurring in a subject which may be predisposed to the disease or condition but has not yet been diagnosed as having it; (b) inhibiting the disease or condition (e.g., arresting its development); or (c) relieving the disease or condition (e.g., causing regression of the disease or condition, providing improvement in one or more symptoms). Improvements in any conditions can be readily assessed according to standard methods and techniques known in the art. The population of subjects treated by the method of the disease includes subjects suffering from the undesirable condition or disease, as well as subjects at risk for development of the condition or disease.

By the term "therapeutically effective dose" or "effective amount" is meant a dose or amount that produces the desired effect for which it is administered. The exact dose or amount will depend on the purpose of the treatment, and will be ascertainable by one skilled in the art using known techniques. The term "therapeutically effective amount" is an amount that is effective to ameliorate a symptom of a disease. A therapeutically effective amount can be a "prophylactically effective amount" as prophylaxis can be considered therapy.

The term "sufficient amount" means an amount sufficient to produce a desired effect.

The term "ameliorating" refer" relates to any therapeutically beneficial result in the treatment of a disease state, e.g., a neurodegenerative disease state, including prophylaxis, lessening in the severity or progression, remission, or cure thereof.

The term "adjunctive/add-on treatment" is used herein as understood by the skilled person. In particular, it encompasses any treatment given to bolster or enhance the effectiveness of a previous treatment, in particular in cases where the first treatment proved not to be fully effective.

The actual amount administered, and rate and time-course of administration, will depend on the nature and severity of disease being treated. Prescription of treatment, e.g. decisions on dosage etc., is within the responsibility of general practitioners and other medical professionals, and typically takes account of the disorder to be treated, the condition of the individual patient, the site of delivery, the method of administration and other factors known to practitioners.

In some embodiments, the patch of the invention is administered to treat a particular primary disease, injury, disorder, or condition. Additionally or alternatively, the patch may be administered to treat pain or anxiety associated with a disease, injury, disorder, or condition. In other embodiments, the patch may be administered to treat symptoms secondary to the primary disease, injury, disorder, or conditions.

The term "preventing" is art-recognized to include administration of a pharmaceutical which reduces the frequency of, or delays the onset of, symptoms of a medical condition in a subject relative to a subject which does not receive the composition. E.g., prevention of symptom burden within a patient, including but not limited to pain or anxiety, includes, for example, reducing the degree of, or delaying, pain or anxiety sensations experienced by subjects in a treated population versus an untreated control population.

In one embodiment the patch according to the invention is used for the treatment or prophylaxis of pain. The pain may be chronic pain or acute pain. Chronic pain or acute pain differ in duration, and underlying mechanism.

Neuropathic pain and cancer pain are subcategories of chronic pain known in the art (Wang and Wang (2003) Advanced Drug Delivery Reviews 55: 949-965). Neuropathic pain results from damage (e.g., from disease, injury, age) to the nervous system (e.g., nerves, spinal cord, CNS, PNS). Cancer-related pain can be related to the particular treatment regimen (e.g., radiation, chemotherapeutics, surgery) undertaken or to tumor infiltration, compression of nerves and substances secreted by tumors.

Pain treated by the patch of the invention may be nociceptive, inflammatory, or neuropathic. Nociceptive pain is pain experienced following, for example, changes or extremes in temperature, exposure to acids, exposure to chemical agents, exposure to force, and exposure to pressure. Inflammation results from the immune system's response to injury or disease and the recruitment of macrophages, mast cells, neutrophils, and other cells of the immune system. Which along with the release of cytokines and other factors (e.g., histamine, serotonin, bradykinin, prostaglandins, ATP, H+, nerve growth factor, TNFa, endothelins, interleukins), can cause fever, swelling, and pain. Standard therapy for the pain of inflammation includes Cox2 inhibitors and opioids. Neuropathic pain results from damage (e.g., from disease, injury, age) to the nervous system- (e.g., nerves, spinal cord, CNS, PNS). Tricyclic antidepressants, anticonvulsants, Na+ channel blockers, NMDA receptor antagonists, and opioids are useful in the treatment of neuropathic pain.

The "treatment or prophylaxis of pain" also encompasses the treatment of pain as one symptom within the symptom burden of patients with severe chronic diseases, such as ALS or cancer. In certain embodiments the pain results from inflammation, including inflammation from multiple sclerosis, ALS, Alzheimer disease or other neurodegenerative diseases, inflammatory diseases, Parkinson's disease, motor neuron diseases and traumatic brain injury. The pain may be acute and/or chronic pain including chronic pain from motor neuron disease, multiple sclerosis, Fibromyalgia, cancer, ALS and peripheral neuropathy.

In one embodiment the pain is acute or chronic pain including pain from motor neuron disease, neurodegeneration diseases, multiple sclerosis, Fibromyalgia, cancer and peripheral neuropathy.

In one embodiment the pain is back pain, including upper/neck, shoulder, cervical and lower back pain, neuropathic pain, cancer-related pain.

In one embodiment the treatment or prophylaxis of pain is in relation to diseases or conditions resulting in pain as a side effect, e.g. in the second or third line, or adjunctive/add-on treatment of cancer or ALS.

In another embodiment, the patch can be used to treat or prevent a condition selected from the group consisting of glaucoma, neuralgia, somatic pain, chronic pain, neuropathic pain, labor pain, inflammation, muscle spasticity (such as that associated with spinal cord injury or multiple sclerosis), movement disorders (such as that associated with dystonia, Parkinson's disease, Huntington's disease, or Tourette's syndrome), migraine headache, epilepsy, ALS, Alzheimer disease or other neurodegenerative diseases, and inflammatory diseases.

In a further embodiment the pain is selected from the group consisting of chronic pain including chronic pain from motor neuron disease, multiple sclerosis, Fibromyalgia, cancer, ALS and peripheral neuropathy, acute pain, neuropathic pain, pain from chronic disease, nociceptive pain, inflammatory pain, including inflammation from multiple sclerosis, Alzheimer disease or other neurodegenerative diseases, Parkinson disease motor neuron disease and traumatic brain injury, neuropathic pain, chemotherapy-induced pain or visceral pain.

The patch according to the invention may also be used to treat anxiety, also in the form of being part of the symptom burden outlined above. In some embodiments, the anxiety treated is related to a patient having a terminal disease, or having a chronic disease. In some embodiments, the anxiety may be generalised anxiety disorder. In some embodiments, the anxiety may be selected from the group consisting of social anxiety, one or more specific phobias, panic disorder, obsessive compulsive disorder, and post-traumatic stress disorder.

### Preparation of the transmucosal patch

The cannabinoid is mixed with a solvent into which it solvates or dissolves. As used herein "to solvate" or "solvation" refers to the process of attraction and association of molecules of a solvent with molecules or ions of a solute, here, a cannabinoid. Solvation is the process of surrounding the solute, or cannabinoid, with solvent. It involves evening out a concentration gradient and evenly distributing the solute within the solvent. The solvent may be a pharmaceutically acceptable solvent. Non-limiting examples of useful solvents are e.g., polyethylene glycols (PEG), propylene glycol, substituted polyethylene glycols, propylene carbonate, ethanol, ethyl acetate, isopropyl alcohol, triacetin, triethyl citrate, tributyl citrate, substituted polyethylene glycols, bisabolol, glycerin, mineral oil, ethyl oleate, oleic acid, fatty acid esters, lactic acid, dipropylene glycol, hexylene glycol, propylene carbonate, benzyl benzoate, benzyl alcohol, perillyl alcohol, dibutyl sebacate, phenyethyl alcohol, n-methyl pyrrolidone, dimethyl sulfoxide, 2- pyrrolidone, squalane, animal oils, vegetable oils, dimethyl isosorbide, hydrogenated vegetable oils, isopropyl myristate, limonene, isopropyl palmitate, glycofurol, terpenes, essential oils, alcohols, diols such as ethanol, polyols, silicone fluids, and combination thereof. One exemplary solvent is the alcohol ethanol. Non- limiting ranges of solvent(s) is from about 0.5% to about 95.0%, about 1% to about 80%, about 5% to about 70%, or about 15% to about 35% weight/weight solvent to cannabinoid.

### Example

For the preparation of the electrospinning solution PVP (13% w/v) and THC/CBD (w/w; ratio 1:1) are dissolved in water/ethanol (1:1 v/v). After overnight dissolution, the solution is vortexted until a homogeneous solution is formed. The solution is briefly degassed in an ultrasonic bath and filled into a Hamilton glass syringe (10 mL), connected via polytetrafluoroethylene (PTFE) Luer lock adapters and PTFE tubing with a stainless steel spinning needle. The solution electrospun at 15 kV with a flow rate of 2 mL/h on a rotating metal drum collector (250 RPM) for 2 hours. The fibers are removed from the collector, wrapped in aluminum foil and stored in a desiccator at 4°C until further use.

## Claims

1. A transmucosal patch comprising electrospun fibers comprising a biocompatible polymer and a therapeutically effective amount of a pharmaceutical active ingredient, wherein the active pharmaceutical ingredient comprises a cannabinoid or a cannabinoid and an opioid.

2. The patch according to claim 1, which is a buccal or sublingual transmucosal patch.

3. The patch according to claim 1 or 2, wherein the active pharmaceutical ingredient is a cannabinoid or a cannabinoid and an opioid.

4. The patch according to any of the preceding claims, which comprises an additional active pharmaceutical ingredient, which is not an opioid or a cannabinoid.

5. The patch according to any of the preceding claims, wherein the active pharmaceutical ingredient is at least one cannabinoid.

6. The patch according to any of the preceding claims, wherein the active pharmaceutical ingredient is a combination of THC and CBD.

7. The patch according to claim 6, wherein the ratio between THC and CBD is 20:1 to 1:20, in particular 1:1.

8. The patch according to any of the preceding claims, wherein the biocompatible polymer is partly or completely dissolvable in contact with saliva.

9. The patch according to any of the preceding claims, wherein the biocompatible polymer stays intact in contact with saliva.

10. The patch according to any of the preceding claims for use as a medicament.

11. The patch according to claim 10 for use in the treatment or prophylaxis of the symptom burden in a patient suffering from a symptoms' generating disease, in particular a severe chronic disease such as ALS or cancer.

12. The patch for use according to claim 11, wherein the symptoms to be treated are selected from pain, depression, restless legs, nausea, constipation, vomiting, decreased or loss of appetite, a sleep disorder, pruritus, anxiety, fatigue, dyspnea, numbness in extremity, dizziness, confusion, memory problems, euphoria, restlessness, somnolence, vomiting, abdominal pain, diarrhea, tachycardia, chest pain, itching, dry mouth, spasms, cramps, respiratory problems, stress, overall reduced activity, reduced quality of life, recurrent infections, inflammation, and a combination of two or more thereof.

13. The patch according to claims 10 to 12 for use in the treatment or prophylaxis of pain.

14. The patch for use according to any of claims 10 to 13, wherein said treatment is a second or third line, or adjunctive/add-on treatment of cancer or ALS.

15. A process for preparing a patch according to any of the preceding claims comprising the following steps (a) to (c):
(a) Providing a solution of a biocompatible polymer;
(b) Providing a liquid comprising the active pharmaceutical ingredient;
(c) Electrospinning said solution of a biocompatible polymer to produce a fiber comprising said polymer and said active pharmaceutical ingredient.

## Patentansprüche

1. Ein transmukosales Pflaster aufweisend elektrogesponnene Fasern aufweisend ein biokompatibles Polymer und eine therapeutisch effektive Menge eines pharmazeutisch aktiven Inhaltsstoffs, wobei der pharmazeutisch aktive Inhaltsstoff ein Cannabinoid oder ein Cannabinoid und ein Opioid aufweist.

2. Das Pflaster nach Anspruch 1, welches ein bukkal oder sublinguales transmukosales Pflaster ist.

3. Das Pflaster nach Anspruch 1 oder 2, wobei der pharmazeutisch aktive Inhaltsstoff ein Cannabinoid oder ein Cannabinoid und ein Opioid ist.

4. Das Pflaster nach irgendeinem der vorangegangenen Ansprüche, welches einen zusätzlichen pharmazeutisch aktiven Inhaltsstoff aufweist, welcher kein Opioid oder Cannabinoid ist.

5. Das Pflaster nach irgendeinem der vorangegangenen Ansprüche, wobei der pharmazeutisch aktive Inhaltsstoff mindestens ein Cannabinoid ist.

6. Das Pflaster nach irgendeinem der vorangegangenen Ansprüche, wobei der pharmazeutisch aktive Inhaltsstoff eine Kombination aus THC und CBD ist.

7. Das Pflaster nach Anspruch 6, wobei das Verhältnis aus THC zu CBD 20:1 bis 1:20 ist, insbesondere 1:1.

8. Das Pflaster nach irgendeinem der vorangegangenen Ansprüche, wobei das biokompatible Polymer teilweise oder vollständig löslich ist bei Kontakt mit Speichel.

9. Das Pflaster nach irgendeinem der vorangegangenen Ansprüche, wobei das biokompatible Polymer intakt bleibt bei Kontakt mit Speichel.

10. Das Pflaster nach irgendeinem der vorangegangenen Ansprüche, zur Verwendung als ein Medikament.

11. Das Pflaster zur Verwendung nach Anspruch 10 bei der Behandlung oder Prophylaxe der Symptombelastung bei einem Patienten leidend an einer Symptome erzeugenden Krankheit, insbesondere einer schweren chronischen Krankheit wie ALS oder Krebs.

12. Das Pflaster zur Verwendung nach Anspruch 11, wobei die zu behandelnden Symptome ausgewählt sind aus Schmerz, Depression, unruhige Beine, Übelkeit, Verstopfung, Erbrechen, verminderter oder fehlender Appetit, Schlafstörungen, Juckreiz, Angst, Müdigkeit, Dyspnoe, Taubheitsgefühl in den Extremitäten, Schwindel, Verwirrung, Gedächtnisprobleme, Euphorie, Unruhe, Schläfrigkeit, Erbrechen, Bauchschmerzen, Durchfall, Tachykardie, Brustschmerzen, Juckreiz, Trockenheit Mund, Spasmen, Krämpfe, Atemprobleme, Stress, insgesamt verminderte Aktivität, verminderte Lebensqualität, wiederkehrende Infektionen, Entzündungen und eine Kombination von zwei oder mehreren davon.

13. Das Pflaster zur Verwendung nach den Ansprüchen 10 bis 12 bei der Behandlung oder Prophylaxe von Schmerz.

14. Das Pflaster zur Verwendung nach den Ansprüchen 10 bis 13, wobei besagte Behandlung eine Zweit- oder Drittlinien-, oder ergänzende/zusätzliche Behandlung von Krebs oder ALS ist.

15. Ein Verfahren zum Herstellen eines Pflasters nach irgendeinem der vorangegangenen Ansprüche aufweisend die folgenden Schritte (a) bis (c):
(a) Bereitstellen einer Lösung eines biokompatiblen Polymers;
(b) Bereitstellen einer Flüssigkeit aufweisend den pharmazeutisch aktiven Inhaltsstoff;
(c) Elektrospinnen besagter Lösung eines biokompatiblen Polymers zum Herstellen einer Faser aufweisend besagtes Polymer und den pharmazeutisch aktiven Inhaltsstoffs.

## Revendications

1. Timbre transmucosal comprenant des fibres électrofilées comprenant un polymère biocompatible et une quantité thérapeutiquement efficace d'un principe actif pharmaceutique, dans lequel le principe actif pharmaceutique comprend un cannabinoïde ou un cannabinoïde et un opioïde.

2. Timbre selon la revendication 1, qui est un timbre transmucosal buccal ou sublingual.

3. Timbre selon la revendication 1 ou 2, dans lequel le principe actif pharmaceutique est un cannabinoïde ou un cannabinoïde et un opioïde.

4. Timbre selon l'une quelconque des revendications précédentes, qui comprend un principe actif pharmaceutique supplémentaire, qui n'est pas un opioïde ni un cannabinoïde.

5. Timbre selon l'une quelconque des revendications précédentes, dans lequel le principe actif pharmaceutique est au moins un cannabinoïde.

6. Timbre selon l'une quelconque des revendications précédentes, dans lequel le principe actif pharmaceutique est une combinaison de THC et de CBD.

7. Timbre selon la revendication 6, dans lequel le rapport entre THC et CBD est de 20 : 1 à 1 : 20, en particulier de 1 : 1.

8. Timbre selon l'une quelconque des revendications précédentes, dans lequel le polymère biocompatible est partiellement ou complètement soluble au contact de la salive.

9. Timbre selon l'une quelconque des revendications précédentes, dans lequel le polymère biocompatible reste intact au contact de la salive.

10. Timbre selon l'une quelconque des revendications précédentes pour son utilisation en tant que médicament.

11. Timbre pour son utilisation selon la revendication 10 dans le traitement ou la prophylaxie du fardeau des symptômes chez un patient souffrant d'une maladie générant des symptômes, en particulier une maladie chronique grave telle que la sclérose latérale amyotrophique SLA ou le cancer.

12. Timbre pour son utilisation selon la revendication 11, dans lequel les symptômes à traiter sont choisis parmi douleur, dépression, jambes sans repos, nausées, constipation, vomissements, diminution ou perte d'appétit, trouble du sommeil, prurit, anxiété, fatigue, dyspnée, engourdissement des extrémités, vertiges, confusion, troubles de la mémoire, euphorie, agitation, somnolence, vomissements, douleurs abdominales, diarrhée, tachycardie, douleurs thoraciques, démangeaisons, sécheresse de la bouche, spasmes, crampes, problèmes respiratoires, stress, réduction générale de l'activité, réduction de la qualité de vie, infections récurrentes, inflammation et une combinaison de deux ou plus de ceux-ci.

13. Timbre destiné pour son utilisation selon les revendications 10 à 12 dans le traitement ou la prophylaxie de la douleur.

14. Timbre pour son utilisation selon l'une quelconque des revendications 10 à 13, dans lequel ledit traitement est un traitement de deuxième ou de troisième ligne, ou un traitement d'appoint/complémentaire du cancer ou de la SLA.

15. Procédé de préparation d'un timbre selon l'une quelconque des revendications précédentes, comprenant les étapes suivantes (a) à (c) :
(a) la fourniture d'une solution d'un polymère biocompatible ;
(b) la fourniture d'un liquide comprenant le principe actif pharmaceutique :
(c) l'électrofilage de ladite solution d'un polymère biocompatible afin de produire une fibre comprenant ledit polymère et ledit principe actif pharmaceutique.
